(19) Europäisches Patentamt
European Patent Office
Office européen des brevets

(11) Veröffentlichungsnummer: **0 008 014**
**A1**

(12) **EUROPÄISCHE PATENTANMELDUNG**

(21) Anmeldenummer: **79102436.7**

(22) Anmeldetag: **13.07.79**

(51) Int. Cl.³: **C 07 D 401/04**, C 07 D 405/14, C 07 D 409/14, C 07 D 491/04, A 61 K 31/445, A 61 K 31/47 // (C07D491/04, 317/00, 221/00)

(30) Priorität: **18.07.78 DE 2831431**
**15.03.79 DE 2910194**

(43) Veröffentlichungstag der Anmeldung: **20.02.80**
**Patentblatt 80/4**

(84) Benannte Vertragsstaaten: **AT BE CH DE FR GB IT NL**

(71) Anmelder: **HOECHST AKTIENGESELLSCHAFT, Zentrale Patentabteilung Postfach 80 03 20, D-6230 Frankfurt/Main 80 (DE)**

(72) Erfinder: **Lattrell, Rudolf, Dr., Heuhohlweg 6H, D-6240 Königstein/Taunus (DE)**
Erfinder: **Bartmann, Wilhelm, Dr., Am Dachsbau 5, D-6232 Bad Soden am Taunus (DE)**
Erfinder: **Kaiser, Joachim, Dr., Egerländer Strasse 8, D-6233 Kelkheim (Taunus) (DE)**

(54) **1-(4-Aminopiperidino)-3,4-dihydroisochinoline, Verfahren zu ihrer Herstellung, pharmazeutische Präparate auf Basis dieser Verbindungen sowie ihre Verwendung.**

(57) 1-(4-Aminopiperidino)-3,4-dihydroisochinoline der Formel

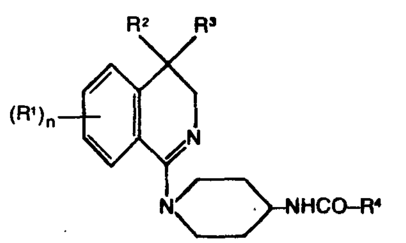

worin R¹, R², R³, R⁴ und n die angegebenen Bedeutungen haben, deren physiologisch verträglichen Salze, Verfahren zur Herstellung dieser Verbindungen und pharmazeutische Präparate, die diese enthalten.

Die Verbindungen besitzen wertvolle pharmakologische, insbesondere Herz-Kreislauf-wirksame Eigenschaften und können daher als Arzneimittel verwendet werden.

EP 0 008 014 A1

# 1-(4-Aminopiperidino)-3,4-dihydroisochinoline, Verfahren zu ihrer Herstellung, pharmazeutische Präparate auf Basis dieser Verbindungen sowie ihre Verwendung

Die Erfindung betrifft neue 1-(4-Aminopiperidino)-3,4-dihydroisochinolinderivate, die wertvolle pharmakologische, insbesondere Herz-Kreislauf-wirksame Eigenschaften besitzen, und daher als Arzneimittel geeignet sind.

Gegenstand der Erfindung sind 1-(4-Aminopiperidino)-3,4-dihydroisochinoline der Formel I

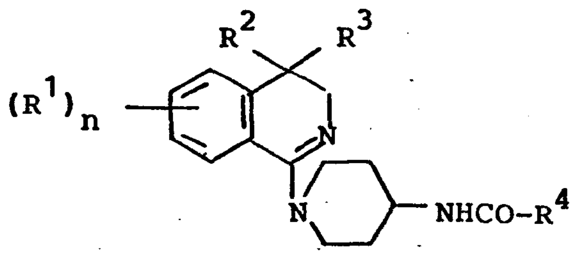

I

worin bedeuten

$R^1$ Wasserstoff, $C_1$-$C_6$-Alkyl, Phenyl, $C_1$-$C_6$-Alkoxy, $C_1$-$C_6$-Alkylthio, Benzyloxy, Methylendioxy, Halogen, Trifluormethyl, Hydroxy, Nitro, Amino, $C_1$-$C_6$-Alkoxycarbonyl, Sulfamoyl, wobei, falls $n > 1$, die $R^1$ gleich oder verschieden sein können

n eine Zahl von 1 bis 3

$R^2$ und $R^3$ gleiche oder verschiedene Substituenten folgender Bedeutung: Wasserstoff, Phenyl, Benzyl oder eine geradkettige oder verzweigte $C_1$-$C_6$-Alkylgruppe,

$R^4$ $C_1$-$C_6$-Alkyl, $C_2$-$C_6$-Alkenyl, $C_1$-$C_6$-Alkyloxy, das gegebenenfalls mit Hydroxy substituiert ist, $C_2$-$C_6$-Alkenyloxy, Cycloalkyl mit 5 bis 7 Kohlenstoffatomen, Cycloalkyloxy mit 5 bis 7 Kohlenstoffatomen, Aryl, Aryl-$C_1$-$C_6$-alkyl, Diaryl-$C_1$-$C_6$-alkyl, Aryl-$C_1$-$C_6$-alkyloxy oder Aryloxy, wobei Aryl für Phenyl-, Furyl-,

Thienyl- oder Pyridyl steht und wobei die Arylgruppen mono-, di- oder trisubstituiert sein können mit $C_1$-$C_6$-Alkyl, $C_1$-$C_6$-Alkoxy, $C_1$-$C_6$Alkylthio, Phenyl, Benzyloxy, Methylendioxy, Halogen, Trifluormethyl, Hydroxy, Nitro, Amino, $C_1$-$C_6$-Alkoxycarbonyl oder Sulfamoyl,

sowie deren physiologisch verträgliche Salze.

Falls der Arylrest di- oder trisubstituiert ist, können die Substituenten gleich oder verschieden sein.

Als bevorzugte Substituenten kommen in Betracht:

Für $R^1$: Wasserstoff, geradkettiges oder verzweigtes $C_1$-$C_4$-Alkyl, Phenyl, Methoxy, Äthoxy, Isopropoxy, n-Butoxy, Methylendioxy, Fluor, Chlor, Brom, Trifluormethyl, Hydroxy, Nitro, Amino

Für n: sind bevorzugt die Zahlen 1 und 2, wobei im Falle der Disubstitution die 5-, 7- und 6- und 7-Stellung bevorzugt ist.

Für $R^2$: Wasserstoff, Phenyl, verzweigtes oder geradkettiges $C_1$-$C_4$-Alkyl.

Für $R^3$: Wasserstoff, Phenyl, Benzyl oder verzweigtes oder geradkettiges $C_1$-$C_4$-Alkyl,

Für $R^4$: geradkettiges oder verzweigtes $C_1$-$C_4$Alkyl bzw. $C_2$-$C_4$-Alkenyl, geradkettiges oder verzweigtes $C_1$-$C_4$Alkyloxy wie Methoxy, Äthoxy, Propyloxy, Isopropyloxy, Isobutyloxy, tert. Butyloxy, mit Hydroxy substituiertes $C_1$-$C_4$-Alkyloxy wie z.B. 2-Hydroxy-2-methylpropyloxy, geradkettiges oder verzweigtes $C_2$-$C_4$Alkenyloxy, wie 2-Methylpropenyloxy, Cyclohexyl,ein unsubstituierter Phenylrest, oder ein Phenylrest, der durch geradkettiges oder verzweigtes $C_1$-$C_4$-Alkyl,

Phenyl, Methoxy, Dimethoxy, 3,4,5-tri-Methoxy, 2,4,5-tri-Methoxy, 3,4-Methylendioxy, Trifluormethyl, Nitro, Amino, Fluor, Chlor, Brom oder Sulfamoyl substituiert ist, oder ein 2- oder 3-Furylrest, ein 2- oder 3-Thienylrest, der durch Chlor und/oder Sulfamoyl substituiert sein kann oder ein 2-, 3- oder 4-Pyridylrest, Benzyl, Diphenylmethyl, Benzyloxy, Phenoxy.

Besonders bevorzugt sind Verbindungen, in welchen bedeuten:

$R^1$ Wasserstoff, Methyl, Phenyl, Methoxy, Fluor, Chlor, Brom oder Methylendioxy,

n 1 oder 2, wobei im Falle der Disubstitution die 5,7 und 6,7-Dichlor- und Dimethoxy-Verbindungen bevorzugt sind,

$R^2$ Wasserstoff oder Alkyl mit bis zu 2 Kohlenstoffatomen,
$R^3$ Wasserstoff, Alkyl mit bis zu 2 Kohlenstoffatomen, Phenyl oder Benzyl und
$R^4$ geradkettiges oder verzweigtes Alkyl oder Alkenyl mit 3 bis 4 Kohlenstoffatomen, geradkettiges oder verzweigtes Alkyloxy mit 1 bis 4 Kohlenstoffatomen, 2-Hydroxy-2-methylpropyloxy, 2-Methyl-propenyloxy, Cyclohexyl, Phenyl, mit Trifluormethyl, Nitro, Amino oder Chlor monosubstituiertes, mit Chlor und Sulfamoyl disubstituiertes oder mit Methoxy trisubstituiertes Phenyl, 2-Thienyl, Benzyl, Diphenylmethyl, Benzyloxy oder Phenyloxy.

Die Verbindungen besitzen wertvolle pharmakologische, insbesondere Herz-Kreislauf wirksame, Eigenschaften und sind als Arzneimittel geeignet. Die Erfindung betrifft daher auch Arzneimittel auf Basis dieser Verbindungen sowie ihre Verwendung bei der Behandlung von Herz-Kreislauf-Krankheiten.

Die Erfindung betrifft weiterhin ein Verfahren zur Herstellung von Verbindungen der Formel I, das dadurch gekennzeichnet ist, daß man

a) ein 1-Alkoxy oder 1-Alkylthio-3,4-dihydroisochinolin der Formel II,

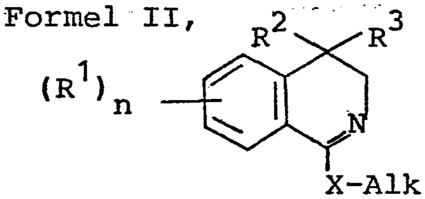

II

worin $R^1$, $R^2$ und $R^3$ die zur Formel I genannte Bedeutung haben und X Sauerstoff oder Schwefel und Alk $C_1$-$C_3$-Alkyl, vorzugsweise Methyl oder Äthyl, bedeuten, mit einem 4-Aminopiperidin der Formel III,

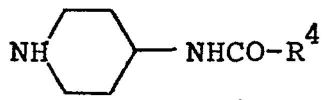

III

worin $R^4$ die zur Formel I genannte Bedeutung hat, umsetzt, oder

b) ein 3,4-Dihydroisochinolinthion der Formel IV,

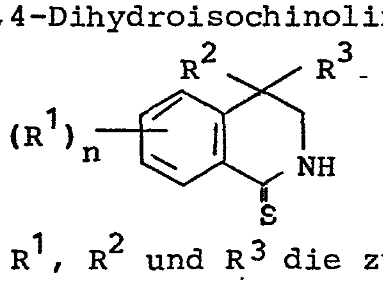

IV

worin $R^1$, $R^2$ und $R^3$ die zur Formel I genannte Bedeutung haben, mit einem 4-Aminopiperidin der Formel III umsetzt, oder

c) eine Verbindung der Formel V

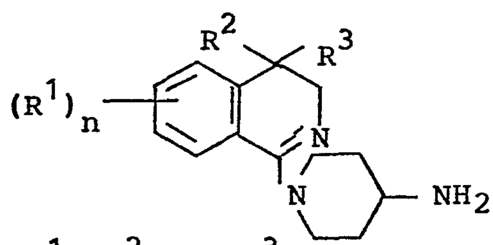

V

worin $R^1$, $R^2$ und $R^3$ die zur Formel I genannte Bedeutung haben, mit einer Carbonsäure der Formel HOOC-$R^4$ oder einem Carbonsäurehalogenid bzw. Halogenkohlensäurederivat der Formel Hal-CO-$R^4$ oder einem Carbonsäureanhydrid der Formel $(R^4CO)_2O$, worin $R^4$ die obengenannte Bedeutung hat und Hal ein Halogenatom bedeutet, umsetzt,

gegebenenfalls eine erhaltene Verbindung, worin $R^4$ einen $C_1$-$C_6$-Alkenylrest bedeutet, in bekannter Weise in eine Verbindung der Formel I, worin $R^4$ einen mit Hydroxy substituierten $C_1$-$C_6$-Alkoxyrest darstellt, überführt, gegebenenfalls bei einer erhaltenen Verbindung, worin $R^4$ einen mit Nitro substituierten Arylrest bedeutet, die Nitrogruppe zur Aminogruppe reduziert und gegebenenfalls die Verbindungen in ihre physiologisch verträglichen Salze überführt.

Nach der Verfahrensvariante a) wird ein 1-Alkylthio-3,4-dihydroisochinolin der Formel II, das in an sich bekannter Weise, z.B. nach den in der DE-OS 1 911 519 und USP 3 644 366 beschriebenen Verfahren, dargestellt werden kann, mit einem 4-Aminopiperidin der Formel III in An- oder Abwesenheit eines Lösungsmittels umgesetzt. Werden Lösungsmittel verwendet, so kommen beispielsweise Äther, wie Dioxan oder Diglym, aromatische Kohlenwasserstoffe, wie Toluol oder Chlorbenzol, Alkohole wie Äthanol oder Isoamylalkohol, aprotische Lösungsmittel wie Dimethylformaid, Dimethylacetamid oder dergleichen in Frage. Die Reaktion wird bei einer Temperatur im Bereich von 30 bis 200°C, vorzugsweise zwischen 60 und 180°C unter Verwendung von vorzugsweise äquimolaren Mengen des Amins der Formel III ausgeführt. Die Reaktion eines 1-Alkoxy-3,4-dihydroisochinolins der Formel II, die in an sich bekannter Weise durch Alkylierung der entsprechenden Amide, z.B. nach der in J. of.Med. Chemistry <u>20</u>, 449 (1977) beschriebenen Methode dargestellt werden, mit einem Amin der Formel III, erfordern im allgemeinen etwas höhere Reaktionstemperaturen und längere Reaktionszeiten im Vergleich zur Reaktion der entsprechenden 1-Alkylthioverbindungen der Formel II. Im Prinzip werden jedoch die beschriebenen Reaktionsbedingungen angewendet.

Nach der Verfahrensvariante b) wird ein Thioamid der Formel IV, das in an sich bekannter Weise z.B. nach den in den DE-OS 1 911 519, 2 143 744 und 2 143 745 beschriebenen Methoden hergestellt werden kann, mit einem Amin der Formel III, wie vorstehend für Verfahren a) beschrieben, umgesetzt.

Die Ausgangsverbindungen V des Verfahrens c) sind neu. Sie können nach den Verfahren a) oder b) aus den Verbindungen der Formel II oder IV und 4-Aminopiperidin hergestellt werden. Vorzugsweise wird jedoch zunächst mit einem geschützten 4-Aminopiperidin umgesetzt. Als Schutzgruppe der 4-Aminogruppe der 4-Aminofunktion können die aus der Peptidchemie bekannten Aminoschutzgruppen, z.B. die tert.-Butyloxycarbonyl-, die p-Methoxybenzyloxycarbonyl-, die Trifluoracetyl-, die 2-Nitro-4-methoxyphenylsulfenylschutzgruppe und dergleichen verwendet werden. In den so erhaltenen Verbindungen der Formel Va

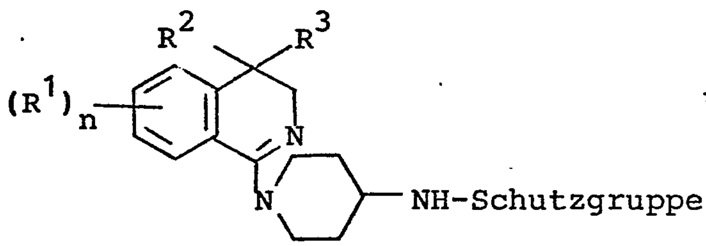

V a

lassen sich die Schutzgruppen nach an sich bekannten Methoden unter Bildung der Verbindungen V abspalten. Vorzugsweise wird die tert.Butyloxycarbonylschutzgruppe verwendet, die quantitativ, z.B. mit Salzsäure/Eisessig oder Trifluoressigsäure, unter Bildung der Verbindungen V abgespalten werden kann. Eine Abspaltung kann in solchen Fällen unterbleiben, in denen die Schutzgruppe einen Rest der Formel $-CO-R^4$ bedeutet.

Die Acylierung der Verbindungen V unter Bildung der Verbindungen I erfolgt nach an sich bekannten Methoden durch Reaktion mit den oben genannten Carbonsäuren $HOOCR^4$, z.B. in Anwesenheit eines wasserabspaltenden Mittels wie Dicyclohexylcarbodiimid, bzw. mit den oben genannten Säurehalogeniden, Säureanhydriden oder Chlorkohlensäureestern der Formel $Cl-CO-R^4$.

Die Verbindungen der Formel I werden in freier Form oder als Salze isoliert, je nach den angewandten Reaktionsbedingungen. Die freien Basen können nach Reaktion mit anorganischen oder organischen Säuren in ihre pharmakologisch verträglichen Salze übergeführt werden. Solche Säuren sind z.B. Salzsäure, Schwefelsäure, Phosphorsäure, aliphatische, alicyclische, araliphatische, aromatische oder heterocyclische Carbonsäuren oder Sulfonsäuren wie Essigsäure, Weinsäure, Milchsäure, Maleinsäure, Fumarsäure, Zitronensäure, Oxalsäure, Methansulfonsäure, Hydroxyäthansulfonsäure oder synthetische Harze, die saure Gruppen enthalten.

Die erfindungsgemäßen Verbindungen sind zur Verwendung als Arzneimittel geeignet. Sie zeigen insbesondere blutdrucksenkende sowie antiarrythmische Eigenschaften. Die gleichen pharmakologischen Eigenschaften zeigen die Zwischenprodukte der Formel V.

a) Die blutdrucksenkende Wirkung wurde am narkotisierten Hund und an der Hochdruckratte bestimmt.

1) Hunde werden mit Pentobarbital-Natrium (z.B. Nembutal (R)) (35 mg/kg i.p.) narkotisiert, die Prüfsubstanz in Dosen von 0,5-5 mg/kg intravenös appliziert und die Blutdrucksenkung fortlaufend über 30 bis 60 Minuten (bei längerer Wirksamkeit auch länger) registriert. Eine signifikante Senkung von mindestens 20 mm über mindestens 30 Minuten tritt nach Verabreichung folgender Verbindungen in folgenden Dosen ein:

| Verbindung gemäß Beispiel Nr. | 1 | 18 | 33d | 33e | 35 |
|---|---|---|---|---|---|
| Dosis mg/kg | 0,5 | 3 | 1 | 1 | 3 |

2) Zur Prüfung an der genetischen Hochdruckratte werden die Substanzen oral verabreicht. Die Verbindung des Beispiels 1 bewirkt mit 10 mg/kg per os innerhalb von 24 Stunden einen Blutdruckabfall von 30 - 60 mm.

b) Zur Prüfung auf antiarrythmische Wirkung werden mit Strophanthin vergiftete Hunde verwendet (Methode: Laszlo Szekeres, Institute of Pharmacology, University Medical

School of Szeged, Hungary. "Methods for Evaluating Antiarrhythmic Agents" in "Methods in Pharmacology I, New York 1971, Herausgeber A. Schwartz).

Hierfür wurden Bastard-Hunde beiderlei Geschlechts in Nembutal-Narkose (35 mg/kg i.p.) mit 4 µg/kg/ml/Min. K-Strophanthin per intravenöser Dauerinfusion bis zum Auftreten gehäufter ventrikulärer Extrasystolen vergiftet. Die Prüfsubstanzen wurden intravenös in 5 % wässriger Lösung verabreicht. Registrierung des EKG (Extremitätenableitungen) und des Blutdruckes auf einem Direktschreiber, gleichzeitige Beobachtung auf einem Oscilloskop. Als wirksam gewertet wurde eine Substanz, die die genannten Rhythmusstörungen völlig zu beseitigen vermochte. Die Verbindungen der Beispiele 11 und 33c beseitigen die Herzrhythmusstörungen sofort nach intravenöser Injektion von je 1 mg/kg.

Die neuen Verbindungen können entweder allein oder mit physiologisch verträglichen Hilfs- oder Trägerstoffen vermischt angewandt werden. Sie können oral,parenteral, i.m. oder i.v. verabreicht werden. Für eine orale Anwendungsform werden die aktiven Verbindungen mit den dafür üblichen Substanzen vermischt und durch übliche Methoden in geeignete Darreichungsformen gebracht, wie Tabletten, Steckkapseln, wäßrige, alkoholische oder ölige Suspensionen oder wäßrige, alkoholische oder ölige Lösungen. Als inerte Träger können z.B. Magnesiumcarbonat, Milchzucker oder Maisstärke unter Zusatz anderer Stoffe wie z.B. Magnesiumstearat verwendet werden. Dabei kann die Zubereitung als Trocken- oder Feuchtgranulat erfolgen. Als ölige Trägerstoffe oder Lösungsmittel kommen besonders pflanzliche und tierische Öle in Betracht wie z.B. Sonnenblumenöl oder Lebertran. Als Lösungsmittel der entsprechenden physiologisch verträglichen Salze der aktiven Verbindungen für eine intravenöse Applikation kommen z.B. in Frage: Wasser, physiologische Kochsalzlösung oder Alkohole wie z.B. Äthanol,Propandiol oder

0008014

Glycerin, daneben auch Zuckerlösungen wie z.B. Glukose- oder Mannitlösungen, oder auch eine Mischung aus den verschiedenen genannten Lösungsmitteln.
Pharmazeutische Präparate können neben den erfindungsgemäßen Verbindungen noch weitere pharmakologisch wirksame Substanzen enthalten.

Die erfindungsgemäßen Verbindungen und ihre pharmakologisch verträglichen Salze sind innerhalb eines breiten Dosierungsbereiches wirksam. Die Menge Wirksubstanz pro Dosierungseinheit bzw. die täglich zu verabreichende Dosis kann daher ebenfalls variieren. Sie hängt ab von der Art der Applikation, vom Zustand, Gewicht usw. des zu Behandelnden. Die tägliche Dosierung liegt bei oraler Applikation vorzugsweise zwischen 50 bis 1000 mg Wirksubstanz, insbesondere zwischen 100 und 500 mg. Eine Dosierungseinheit wie z.B. eine Tablette enthält vorzugsweise 50 bis 250 mg. Für die i.v. oder i.m. Anwendung beträgt die tägliche Dosis vorzugsweise 10 bis 200 mg, insbesondere 20 bis 100 mg.

Die Erfindung wird durch die folgenden Beispiele näher erläutert, jedoch nicht beschränkt.

Beispiel 1:

1-(4-Benzamido-1-piperidyl)-3,4-dihydroisochinolin

Verfahren a):

Ein Gemisch aus 8,8 g (0,05 Mol) 1-Methylthio-3,4-dihydroisochinolin und 10,2 g (0,05 Mol) 4-Benzamidopiperidin wird 4 Stunden auf 140°C erhitzt. Der Rückstand wird mit 50 ml Äther digeriert, die Kristalle abgesaugt und mit Äther gewaschen.
Ausbeute 13,3 g (80 % d. Th.) Schmp. 183°C.

Die Base wird in wenig Methylendichlorid gelöst, mit einem geringen Überschuß an äthanolischer Salzsäure versetzt und zweimal mit Aceton abgedampft. Der kristalline Rückstand wird mit Aceton digeriert, abgesaugt und mit Aceton gewaschen. Man erhält das Hydrochlorid vom Schmp. 258°C.
Ausbeute: quantitativ.

Verfahren b):

1,63 g (0,01 Mol) 3,4-Dihydro-1(2H)-isochinolinthion und 2,1 g (0,01 Mol) 4-Benzamidopiperidin werden in 5 ml N'N-Dimethylformamid 30 Stunden auf 130°C erwärmt. Das Lösungsmittel wird im Vakuum entfernt, der Rückstand in Aceton gelöst und die Titelverbindung wie vorstehend als Hydrochlorid isoliert.
Ausbeute 30 % d.Th., Schmp. 256 - 258°C.

Verfahren α):

Ein Gemisch aus 3,5 g (0,02 Mol) 1-Äthoxy-3,4-dihydroisochinolin, 4,1 g (0,02 Mol) 4-Benzamidopiperidin und 30 ml Diglym wird 17 Stunden unter Rückfluß erhitzt. Das Lösungsmittel wird im Vakuum entfernt, der ölige Rückstand in 40 ml Äther gelöst. Nach Anreiben bildet sich ein Niederschlag, der abgesaugt und mit Äther gewaschen wird.

Ausbeute 4,3 (67 % d.Th.), Schmp. 182 - 183°C.

Die Verbindungen der folgenden Tabelle werden in Analogie zu Beispiel 1 nach den dort angegebenen Verfahren dargestellt.

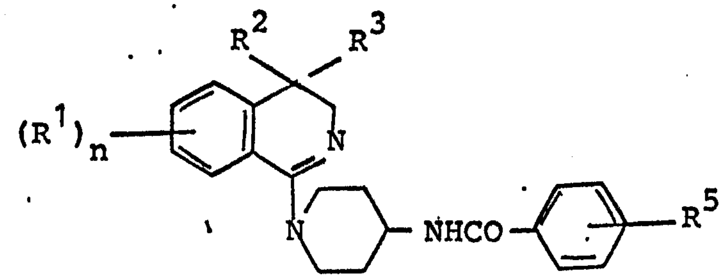

| Beispiel Nr. | $R^1$ | $R^2$ | $R^3$ | $R^5$ | Base Schmp. °C | Hydrochlorid |
|---|---|---|---|---|---|---|
| 2 | H | H | H | 3,4,5-tri-$CH_3O$ | amorph | 140 - 142 |
| 3 | H | H | H | 2,4,5-tri-$CH_3O$ | 180 - 182 | 230 - 232 |
| 4 | H | H | H | 3,4-Methylendioxy | 235 | 208 - 210 |
| 5 | H | H | H | 3-$CF_3$ | amorph | 258 - 260 |
| 6 | H | H | H | 3-$NO_2$ | amorph | 220 - 222 |
| 7 | 6,7-Di-$CH_3O$ | H | H | H | 205 | 105 - 107 |
| 8 | 6,7-Di-$CH_3O$ | H | H | 3-$NO_2$ | 192 - 193 | 288 - 290 |
| 9 | 6,7-Methylendioxy | H | H | H | 198 | 143 - 145 |
| 10 | 6,7-Methylendioxy | H | H | 3,4,5-tri-$CH_3O$ | 235 - 236 | 274 - 276 |
| 11 | 6,7- Methylendioxy | H | H | 3-$CF_3$ | 171 | 276 - 278 |
| 12 | 7-$CH_3O$ | H | H | H | amorph | 240 - 241 |
| 13 | 7-Cl | H | H | H | 199 | 150 - 153 |
| 14 | 7-Cl | H | H | 3,4,5-tri-$CH_3O$ | 202 | 100 - 103 |
| 15 | 7-Cl | H | H | 3-$CF_3$ | 170 | 246 - 248 |

| Beispiel-Nr. | $R^1$ | $R^2$ | $R^3$ | $R^5$ | Base | Hydrochlorid |
|---|---|---|---|---|---|---|
| | | | | | Schmp. °C | |
| 16 | 5-Cl | H | H | H | 161 | 173 - 175 |
| 17 | 5,7-Di-Cl | H | H | H | 194 | 210 - 212 |
| 18 | 6,7-Di-Cl | H | H | H | 220 | 268 - 270 |
| 19 | 7-Br | H | H | H | 194 - 195 | 275 - 277 |
| 20 | 7-F | H | H | H | 204 | 262 - 264 |
| 21 | 5-$CH_3$ | H | H | H | 200 - 202 | 171 - 172 |
| 22 | 7-$CH_3$ | H | H | H | 178 - 180 | 251 - 253 |
| 23 | 7-$C_6H_5$ | H | H | H | 283 - 285 | 168 - 170 |
| 24 | H | H | $C_6H_5$ | H | amorph | 260 - 262 |
| 25 | H | H | $C_6H_5$ | 3,4,5-tri-$CH_3O$ | amorph | 173 - 175 |
| 26 | H | H | $C_6H_5CH_2$ | H | amorph | 210 - 212 |
| 27 | 6-$CH_3O$ | $C_2H_5$ | $C_2H_5$ | H | amorph | 206 - 208 |
| 28 | H | $C_2H_5$ | $C_6H_5$ | H | amorph | 198 - 200 |
| 29 | H | $C_2H_5$ | $C_6H_5$ | 3-$CF_3$ | amorph | 237 - 239 |
| 30 | H | $C_2H_5$ | $C_6H_5$ | 3-$NO_2$ | amorph | 169 - 171 |

Die in den Beispielen 1 und 6 verwendeten Ausgangsverbindungen der allgemeinen Formel III werden wie folgt dargestellt:

4-Benzamidopiperidin:
4-Amino-1-benzyl-piperidin wird mit Benzoylchlorid in üblicher Weise zum 4-Benzamido-1-benzyl-piperidin vom Schmp. 172°C acyliert.

88 g (0,3 Mol) dieser Verbindung werden in einem Gemisch aus 500 ml Eisessig und 150 ml Wasser nach Zusatz von 10 g 10 proz. Palladium/Kohle in der Schüttelapparatur bei 50°C hydriert. Vom Katalysator wird filtriert, das Lösungsmittel im Vakuum entfernt, der Rückstand mit konz. Natronlauge stark alkalisch gestellt und mit Methylendichlorid extrahiert. Die organische Phase wird mit Kaliumcarbonat getrocknet, das Lösungsmittel verdampft. Der kristalline farblose Rückstand ist reines 4-Benzamidopiperidin vom Schmp. 141 - 142°C.
Ausbeute 90 % d. Th.
Die in den übrigen Beispielen verwendeten substituierten 4-Benzamido-piperidinderivate werden in analoger Weise ausgehend vom 4-Amino-1-benzyl-piperidin und den entsprechenden substituierten Benzoylchloriden dargestellt.

4-(3-Nitrobenzamido)piperidin
20,4 g (0,1 Mol) 4-Benzamidopiperidin werden portionsweise in ein Gemisch aus Salpetersäure (D = 1,5) und konz. Schwefelsäure so eingetragen, daß die Temperatur +5°C nicht übersteigt. Die Mischung wird 20 Stunden bei Raumtemperatur belassen, sodann auf 60 g Eis gegossen und portionsweise mit 150 g Kaliumcarbonat versetzt. Das Salzgemisch wird mit insgesamt 1 l Methylendichlorid extrahiert, der Rückstand der organischen Phase mit Äthylacetat digeriert, wobei die Titelverbindung in Lösung geht. Von wenig Ungelöstem wird filtriert, das Lösungsmittel entfernt. Der kristalline Rückstand wird

in Äther suspendiert, abgesaugt und mit Äther gewaschen. Ausbeute 19 g (77 % d.Th.), Schmp. 154 - 156°C.

Beispiel 31:

1-[4-(3-Aminobenzamido)-1-piperidyl]-3,4-dihydroisochinolin

2,1 g (5 mMol) 1-[4-(3-Nitrobenzamido)-1-piperidyl]-3,4-dihydroisochinolin-Hydrochlorid (Beispiel 6) werden in 40 ml Methanol gelöst und nach Zugabe von 0,5 g Raney-Nickel in einer Schüttelapparatur bei Raumtemperatur hydriert. Nach beendeter Wasserstoffaufnahme wird vom Katalysator abgesaugt, das Methanol im Vakuum entfernt, der kristalline Rückstand mit wenig Aceton digeriert, abgesaugt und mit Aceton gewaschen.
Ausbeute 1,72 g (90 % d.Th.) Monohydrochlorid, Schmp. 200 - 202°C/Zersetzung.

Beispiel 32:

1-[4-(3-Aminobenzamido)-1-piperidyl]-6,7-dimethoxy-3,4-dihydroisochinolin-Hydrochlorid

Wie vorstehend aus der Nitroverbindung, Beispiel 8, in 90 % Ausbeute erhalten.
Schmp. 145 - 147°C/Zersetzung.

Beispiel 33

Verfahren c)

1-[4-(4-Chlorbenzamido)-1-piperidyl]-3,4-dihydroisochinolin

a) 1-Benzyl-4-tert.butyloxycarbonylamino-piperidin:
Ein Gemisch aus 28,5 g (0,15 Mol) 4-Amino-1-benzylpiperidin, 21 ml Triäthylamin, 375 ml N,N-Dimethylformamid und 36 g (0,165 Mol) Di-tert.Butyl-dicarbonat wird bei Raumtemperatur bis zur Beendigung der $CO_2$-Entwicklung gerührt. Das Lösungsmittel wird im Vakuum entfernt, der kristalline Rückstand mit Äther verrieben,

abgesaugt und mit Äther gewaschen.
Ausbeute 40 g (92 % d.Th.), Schmp. 123°C.

b) 4-Tert.-butyloxycarbonylamino-piperidin

39,5 (0,135 Mol) der vorstehenden Benzylverbindung in 400 ml Methanol und 20 ml Wasser wird nach Zugabe von 4 g 10 proz. Palladium/Kohle in einer Schüttelapparatur bei Raumtemperatur hydriert. Nach Beendigung der Wasserstoffaufnahme wird vom Katalysator filtriert, das Lösungsmittel im Vakuum entfernt. Der kristalline Rückstand wird mit Äther verrieben.
Ausbeute 25,5 g (84 % d.Th.) farblose Kristalle vom Schmp. 163°C

c) 1-(4-Tert.butyloxycarbonylamino-1-piperidyl)-3,4-dihydroisochinolin

Ein Gemisch aus 17,7 g (0,1 Mol) 1-Methylthio-3,4-dihydroisochinolin und 20 g (0,1 Mol) 4-Tert.butyloxycarbonyl-amino-piperidin wird 7 Stunden auf 150°C erhitzt. Der gelbbraune harzige Rückstand wird über Kieselgel 60 (0,063 - 0,2 mm, Merck), das mit 10 % Wasser desaktiviert ist, mit Chloroform : Methanol : konz. Ammoniak (100 : 10 : 1) filtriert.
Nach nicht umgesetztem 1-Methylthio-3,4-dihydroisochinolin wird die Titelverbindung in Form eines farblosen Harzes eluiert, das nach längerem Stehen kristallisiert. Schmp. 56-58°C.
Ausbeute: 25,8 (94 % d.Th. bezogen auf umgesetztes Ausgangsmaterial)
Hydrochlorid: Schmp. 210 - 212°C

d) 1-(4-Amino-1-piperidyl)-3,4-dihydroisochinolin

7,32 g (0,02 Mol) 1-(4-Tert.butyloxycarbonylamino-1-piperidyl)-3,4-dihydroisochinolin-Hydrochlorid werden in 50 ml Trifluoressigsäure gelöst. Durch Kühlen mit Eiswasser wird die Temperatur zwischen 25 und 35°C gehalten. Nach 30 Minuten wird überschüssige Trifluoressigsäure im

Vakuum entfernt, der Rückstand mit Eis und überschüssiger 4 N Natronlauge verrührt. Die Mischung wird 4 x mit Methylendichlorid extrahiert, die organische Phase mit Magnesiumsulfat getrocknet. Nach Entfernung des Lösungsmittels verbleibt die Titelverbindung als Harz.
Ausbeute: 4,2 g (92 % d. Th.).

Das Dihydrochlorid wird in Analogie zu Beispiel 1 hergestellt: Es schmilzt unter Zersetzung bei 190 - 193°C.

e) 1-/4-(4-Chlorbenzamido)-1-piperidyl7-3,4-dihydroisochinolin

Ein Gemisch aus 2,3 g (0,01 Mol) 1-(4-Amino-1-piperidyl)-3,4-dihydroisochinolin, 2,1 g (0,012 Mol) 4-Chlorbenzoylchlorid, 4 ml Pyridin und 30 ml Chloroform wird 5 Stunden bei Raumtemperatur belassen. Die Mischung wird mit Methylendichlorid verdünnt, mit wäßriger Sodalösung gewaschen, das Lösungsmittel im Vakuum entfernt. Der kristalline Rückstand wird mit Äther verrieben, abgesaugt und mit Äther gewaschen.
Ausbeute: 2.95 g (80 % d.Th.), Schmp. 216°C

Das Hydrochlorird wird in Analogie zu Beispiel 1 hergestellt: Schmelzpunkt 260 - 263°C unter Zersetzung.

Beispiel 34:

1-/4-(2-Thenoylamido)-1-piperidyl7-3,4-dihydroisochinolin

Ein Gemisch aus 2,3 g (0.01 Mol) 1-(4-Amino-1-piperidyl)-3,4-dihydroisochinolin, 30 ml Chloroform, 5 ml Pyridin und 2 g (0,014 Mol) 2-Thenoylchlorid wird 2 Stunden bei Raumtemperatur belassen. Es wird mit Methylendichlorid verdünnt, dreimal mit Wasser gewaschen und das Lösungsmittel im Vakuum entfernt. Der Rückstand wird mit Äther verrieben, der Niederschlag abgesaugt und mit Äther gewaschen.

Ausbeute 2.6 g (77 % d.Th.), Schmp. 108°C

Das Hydrochlorid wird in Analogie zu Beispiel 1 hergestellt. Schmelzpunkt 275 - 276°C.

Beispiel 35

1-(4-Äthyloxycarbonylamino-1-piperidyl)-3,4-dihydroisochinolin

Ein Gemisch aus 2,29 g (0,01 Mol) 1-(4-Amino-1-piperidyl)-3,4-dihydroisochinolin, 2,16 g (0,02 Mol) Chlorameisensäureäthylester, 10 g Kaliumcarbonat und 25 ml Toluol wird 18 Stunden bei Raumtemperatur gerührt. Nach Zugabe von 100 ml Methylendichlorid wird 4x mit Wasser gewaschen, das Lösungsmittel i. Vak. entfernt und der Rückstand mit Diäthyläther digeriert. Der kristalline Niederschlag der Titelverbindung wird abgesaugt und mit Äther gewaschen.

Ausbeute 2,5 g (83 % d. Th.), Schmp. 122 - 124°C

Hydrochlorid: Schmp. 127 - 129°C unter Zersetzung

Die Verbindungen der folgenden Tabelle werden in Analogie zu Beispiel35 aus 1-(4-Amino-1-piperidyl)-3,4-dihydroisochinolin und der entsprechenden Verbindung $ClCOR^4$ dargestellt.

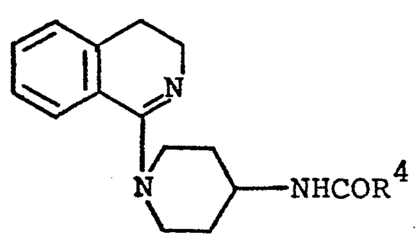

| Beispiel Nr. | $R^4$ | Base Schmp. °C | Hydrochlorid Schmp. °C |
|---|---|---|---|
| 36 | $CH_3O-$ | amorph | >95 |
| 37 | $(CH_3)_2CHO-$ | amorph | 200-202 |
| 38 | $CH_2=C(CH_3)CH_2O-$ | amorph | 191-193 |
| 39 | $C_6H_5O-$ | amorph | 140-142 |
| 40 | $C_6H_5\ CH_2O-$ | Harz | 105-107 |
| 41 | $(CH_3)_2C\ =CH-$ | Harz | 171-173 |
| 42 | $(C_6H_5)_2CH-$ | 185 - 187 | 163-165 |
| 43 | 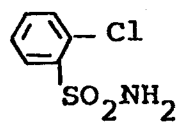 | 265 - 269 (Zers.) | > 280 (Zers.) |

Beispiel 44

6,7-Dimethoxy-1-(4-tert.butyloxy-carbonylamino-1-piperidyl)-3,4-dihydroisochinolin

Ein Gemisch aus 11,86 g (0,05 Mol) 6,7-Dimethoxy-1-methylthio-3,4-dihydroisochinolin, 10 g (0,05 Mol) 4-Tert.butyloxy-carbonylaminopiperidin und 50 ml Dioxan werden 16 Stunden unter Rückfluß erhitzt. Nach dem Erkalten wird mit 50 ml Diäthyläther verdünnt, von nicht umgesetztem 4-Tertbutyloxycarbonyl-aminopiperidin (1,5 g) abgesaugt, die Mutterlauge über Kieselgel 60 (0,063 - 0,2 mm, Merck), das mit 10 % Wasser desaktiviert ist, filtriert. Zunächst wird mit Äthylacetat nicht umsetztes 6,7-Dimethoxy-1-methylthio-3,4-dihydroisochinolin (5,9 g) eluiert, sodann die Titelverbindung mit Chloroform:Methanol:konz. Ammoniak (100:10:1). Ausbeute 7,0 g (72 % d.Th. bezogen auf eingesetztes Ausgangsmaterial) Schmp. 148 - 150°C
Hydrochlorid Schmp. 232 - 234°C unter Zersetzung.

Die Verbindungen der folgenden Tabelle werden in Analogie zu Beispiel 44 aus 1-Methylthio-3,4-dihydro-isochinolin und den entsprechenden Piperidinderivaten 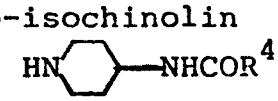 in Dioxan als Lösungsmittel hergestellt.

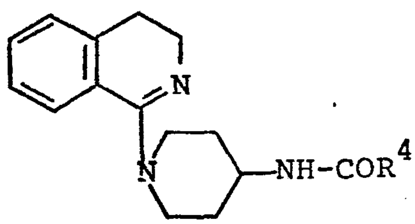

| Beispiel Nr. | $R^4$ | Base Schmp. °C | Hydrochlorid |
|---|---|---|---|
| 45 | Cyclohexyl- | 213 - 215 | 260 - 262 |
| 46 | $CH_3(CH_2)_2-$ | 147 - 149 | 235 - 237 |
| 47 | $C_6H_5CH_2-$ | 165 - 167 | >120 Zersetzung |

Beispiel 48

1-/4-(2-Hydroxy-2-methylpropyl)oxycarbonylamino-1-piperidyl_7-3,4-dihydroisochinolin

3,27 g (0,01 Mol) 1-/4-(2-Methyl-2-propenyl)oxycarbonylamino-1-piperidyl_7-3,4-dihydroisochinolin (Beispiel 38) werden in 30 ml 60 proz. Schwefelsäure gelöst und 18 Stunden bei Raumtemperatur stehengelassen. Die Mischung wird mit konz. wässriger Sodalösung alkalisch gestellt und 4x mit je 50 ml Methylendichlorid extrahiert. Der Rückstand der org. Phase wird in Äther gelöst. Die abgeschiedenen Kristalle der Titelverbindung werden nach 24 Stunden abgesaugt, mit Äther gewaschen und getrocknet.

Ausbeute 2,3 g (66 % d.Th.), Schmp. 135 - 136°C
Hydrochlorid: Schmp. 225-227°C

Patentansprüche:

1. Verbindungen der Formel I

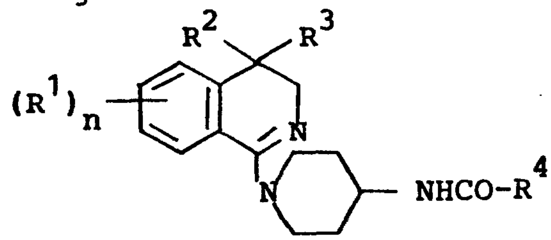

I

worin bedeuten

$R^1$ Wasserstoff, $C_1$-$C_6$-Alkyl, Phenyl, $C_1$-$C_6$-Alkoxy, $C_1$-$C_6$-Alkylthio, Benzyloxy, Methylendioxy, Halogen, Trifluormethyl, Hydroxy, Nitro, Amino, $C_1$-$C_6$-Alkoxycarbonyl, Sulfamoyl, wobei, falls > 1, die $R^1$ gleich oder verschieden sein können

n eine Zahl von 1 bis 3

$R^2$ und $R^3$ gleiche oder verschiedene Substituenten folgender Bedeutung: Wasserstoff, Phenyl, Benzyl oder eine geradkettige oder verzweigte $C_1$-$C_6$-Alkylgruppe,

$R^4$ $C_1$-$C_6$-Alkyl, $C_2$-$C_6$-Alkenyl, $C_1$-$C_6$-Alkyloxy, das gegebenenfalls mit Hydroxy substituiert ist, $C_2$-$C_6$ Alkenyloxy, Cycloalkyl mit 5 bis 7 Kohlenstoffatomen, Cycloalkyloxy mit 5 bis 7 Kohlenstoffatomen, Aryl, Aryl-$C_1$-$C_6$-alkyl, Diaryl-$C_1$-$C_6$-alkyl, Aryl-$C_1$-$C_6$-alkyloxy oder Aryloxy, wobei Aryl für Phenyl-, Furyl-, Thienyl- oder Pyridyl steht und wobei die Arylgruppen mono- di- oder trisubstituiert sein können mit $C_1$-$C_6$-Alkyl, $C_1$-$C_6$Alkoxy, $C_1$-$C_6$Alkylthio, Phenyl, Benzyloxy, Methylendioxy, Halogen, Trifluormethyl, Hydroxy, Nitro, Amino, $C_1$-$C_6$-Alkoxycarbonyl oder Sulfamoyl

sowie deren physiologisch verträgliche Salze.

2. Verbindungen der Formel V

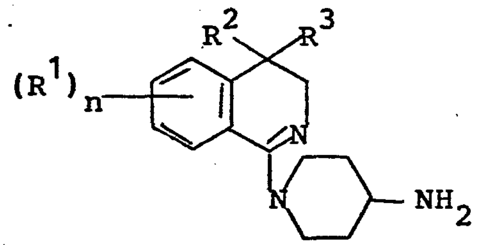

V

worin $R^1$, n, $R^2$ und $R^3$ die zur Formel I in Anspruch 1 genannte Bedeutung haben.

3. Verfahren zur Herstellung von Verbindungen der Formel I, dadurch gekennzeichnet, daß man

a) ein 1-Alkyloxy- oder 1-Alkylthio-3,4-dihydroisochinolin der Formel II,

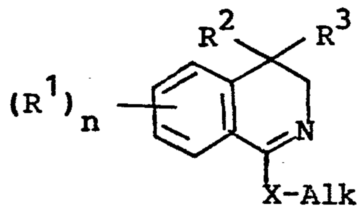

II

worin $R^1$, n, $R^2$ und $R^3$ die zur Formel I genannte Bedeutung haben und X Sauerstoff oder Schwefel und Alk $C_1$-$C_3$-Alkyl bedeuten, mit einem 4-Aminopiperidin der Formel III, 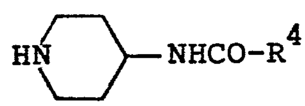 III

worin $R^4$ die zur Formel I genannten Bedeutung hat, umsetzt, oder

b) ein 3,4-Dihydroisochinolinthio der Formel IV,

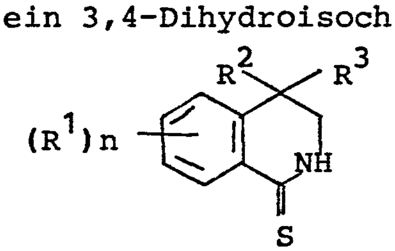

IV

worin, $R^1$, n, $R^2$ und $R^3$ die zur Formel I genannte Bedeutung haben, mit einem 4-Aminopiperidin der Formel III umsetzt, oder

c) eine Verbindung der Formel V,

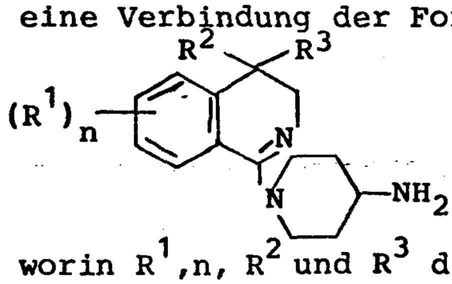

V

worin $R^1$,n, $R^2$ und $R^3$ die zur Formel I genannte Bedeutung haben, mit einer Carbonsäure der Formel HOOC-$R^4$ oder einem Carbonsäurehalogenid bzw. Halogenkohlensäurederivat der Formel Hal-CO-$R^4$ oder einem Carbonsäureanhydrid der Formel $(R^4CO)_2O$ worin $R^4$ die obengenannte Bedeutung hat und Hal ein Halogenatom bedeutet, umsetzt,

gegebenenfalls eine erhaltene Verbindung, worin $R^4$ einen $C_2$-$C_6$-Alkenylrest bedeutet, in bekannter Weise in eine Verbindung der Formel I, worin $R^4$ einen mit Hydroxy substituierten $C_2$-$C_6$-Alkoxyrest darstellt, überführt, gegebenenfalls bei einer erhaltenen Verbindung, worin $R^4$ einen mit Nitro substituierten Arylrest bedeutet, die Nitrogruppe zur Aminogruppe reduziert und gegebenenfalls die Verbindungen in ihre physiologisch verträglichen Salze überführt.

4. Verfahren zur Herstellung von Arzneimitteln, dadurch gekennzeichnet, daß man eine Verbindung gemäß Anspruch 1, gegebenenfalls mit üblichen pharmazeutischen Trägern, und/oder Stabilisatoren, in eine therapeutisch geeignete Darreichungsform bringt.

5. Arzneimittel, gekennzeichnet durch einen Gehalt an einer Verbindung gemäß Anspruch 1 oder bestehend aus einer solchen Verbindung.

6. Verwendung einer Verbindung gemäß Anspruch 1 bei der Behandlung von Herz-Kreislauf-Krankheiten.

7. Verfahren zur Behandlung von Herz-Kreislauf-Krankheiten, dadurch gekennzeichnet, daß man eine wirksame Menge einer Verbindung gemäß Anspruch 1 verabreicht.

Patentansprüche für Österreich:

1. Verfahren zur Herstellung von Verbindungen der Formel I,

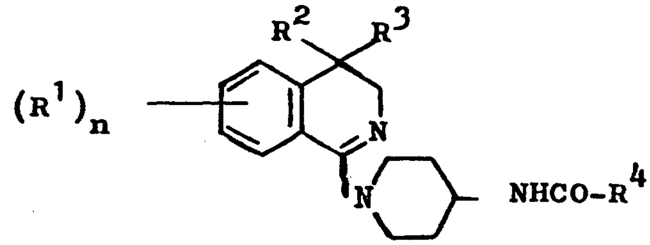

I

worin bedeuten

$R^1$ Wasserstoff, $C_1$-$C_6$-Alkyl, Phenyl, $C_1$-$C_6$-Alkoxy, $C_1$-$C_6$-Alkylthio, Benzyloxy, Methylendioxy, Halogen, Trifluormethyl, Hydroxy, Nitro, Amino, $C_1$-$C_6$-Alkoxycarbonyl, Sulfamoyl, wobei, falls >1, die $R^1$ gleich oder verschieden sein können

n eine Zahl von 1 bis 3

$R^2$ und $R^3$ gleiche oder verschiedene Substituenten folgender Bedeutung: Wasserstoff, Phenyl, Benzyl oder eine geradkettige oder verzweigte $C_1$-$C_6$-Alkylgruppe,

$R^4$ $C_1$-$C_6$-Alkyl, $C_2$-$C_6$-Alkenyl, $C_1$-$C_6$-Alkyloxy, das gegebenenfalls mit Hydroxy substituiert ist, $C_2$-$C_6$ Alkenyloxy, Cycloalkyl mit 5 bis 7 Kohlenstoffatomen, Cycloalkyloxy mit 5 bis 7 Kohlenstoffatomen, Aryl, Aryl-$C_1$-$C_6$-alkyl, Diaryl-$C_1$-$C_6$-alkyl, Aryl-$C_1$-$C_6$-alkyloxy oder Aryloxy, wobei Aryl für Phenyl-, Furyl-, Thienyl- oder Pyridyl steht und wobei die Arylgruppen mono- di oder trisubstituiert sein können mit $C_1$-$C_6$-Alkyl, $C_1$-$C_6$-Alkoxy, $C_1$-$C_6$Alkylthio, Phenyl, Benzyloxy, Methylendioxy, Halogen, Trifluormethyl, Hydroxy, Nitro, Amino, $C_1$-$C_6$-Alkoxycarbonyl oder Sulfamoyl

sowie deren physiologisch verträglichen Salzen

dadurch gekennzeichnet, daß man

a) ein 1-Alkyloxy- oder 1-Alkylthio-3,4-dihydroisochinolin der Formel II,

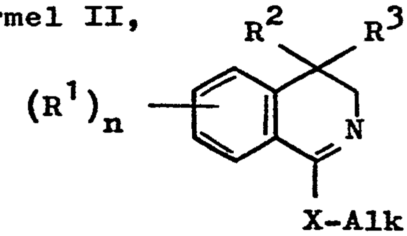

II

worin $R^1$, n, $R^2$ und $R^3$ die zur Formel I genannte Bedeutung haben und X Sauerstoff oder Schwefel und Alk $C_1$-$C_3$-Alkyl bedeuten, mit einem 4-Aminopiperidin der Formel III,

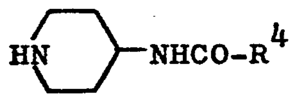

III

worin $R^4$ die zur Formel I genannten Bedeutung hat, umsetzt, oder

b) ein 3,4-Dihydroisochinolinthio der Formel IV,

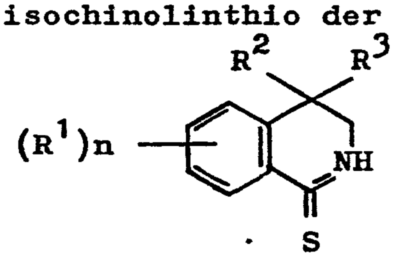

IV

worin, $R^1$, n, $R^2$ und $R^3$ die zur Formel I genannte Bedeutung haben, mit einem 4-Aminopiperidin der Formel III umsetzt, oder

c) eine Verbindung der Formel V,

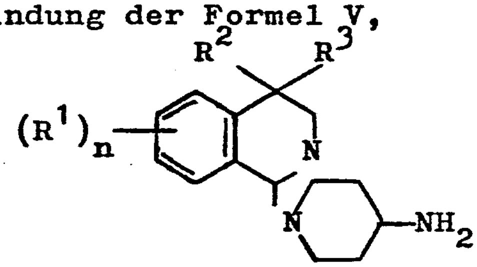

V

worin $R^1$, n, $R^2$ und $R^3$ die zur Formel I genannte Bedeutung haben, mit einer Carbonsäure der Formel HOOC-$R^4$ oder einem Carbonsäurehalogenid bzw. Halogenkohlensäurederivat der Formel Hal-CO-$R^4$

oder einem Carbonsäureanhydrid der Formel $(R^4CO)_2O$ worin $R^4$ die obengenannte Bedeutung hat und Hal ein Halogenatom bedeutet, umsetzt,

gegebenenfalls eine erhaltene Verbindung, worin $R^4$ einen $C_2$-$C_6$-Alkenylrest bedeutet, in bekannter Weise in eine Verbindung der Formel I, worin $R^4$ einen mit Hydroxy substituierten $C_2$-$C_6$-Alkoxyrest darstellt, überführt, gegebenenfalls bei einer erhaltenen Verbindung, worin $R^4$ einen mit Nitro substituierten Arylrest bedeutet, die Nitrogruppe zur Aminogruppe reduziert und gegebenenfalls die Verbindungen in ihre physiologisch verträglichen Salze überführt.

2. Verfahren nach Anspruch 1, d. h. daß Verbindungen der Formel V

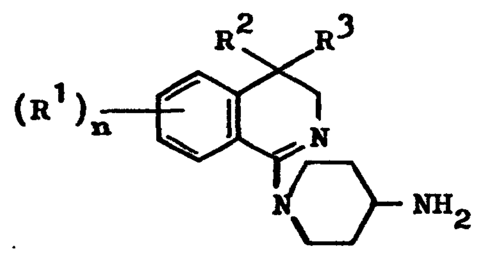

V

worin $R^1$ , n, $R^2$ und $R^3$ die zur Formel I in Anspruch 1 genannte Bedeutung haben, hergestellt werden.

3. Verfahren zur Herstellung von Arzneimitteln, dadurch gekennzeichnet, daß man eine Verbindung gemäß Anspruch 1, gegebenenfalls mit üblichen pharmazeutischen Trägern, und/oder Stabilisatoren, in eine therapeutisch geeignete Darreichungsform bringt.

4. Arzneimittel, gekennzeichnet durch einen Gehalt an einer Verbindung gemäß Anspruch 1 oder bestehend aus einer solchen Verbindung.

5. Verwendung einer Verbindung gemäß Anspruch 1 bei der Behandlung von Herz-Kreislauf-Krankheiten.

6. Verfahren zur Behandlung von Herz-Kreislauf-Krankheiten, dadurch gekennzeichnet, daß man eine wirksame Menge einer Verbindung gemäß Anspruch 1 verabreicht.

0008014
Nummer der Anmeldung
EP 79 102 436.7

Europäisches Patentamt

# EUROPÄISCHER TEILRECHERCHENBERICHT,
der nach Regel 45 des Europäischen Patentübereinkommens für das weitere Verfahren als europäischer Recherchenbericht gilt

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 1 911 464 (RHONE-POULENC)<br>* Ansprüche 1, 5 *<br>-- | 1-3 |
| | DE - A - 1 911 519 (ASPRO-NICHOLAS)<br>* Beispiel 15 *<br>-- | 3 |
| A | DE - A1 - 2 352 702 (HOECHST)<br>---- | |

**KLASSIFIKATION DER ANMELDUNG (Int.Cl.3)**

C 07 D 401/04
C 07 D 405/14
C 07 D 409/14
C 07 D 491/04
A 61 K 31/445
A 61 K 31/47//
(C 07 D 491/04, 317/00, 221/00)

**RECHERCHIERTE SACHGEBIETE (Int. Cl.3)**

A 61 K 31/445
A 61 K 31/47
C 07 D 401/04
C 07 D 405/14
C 07 D 409/14
C 07 D 491/04

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-5
Unvollständig recherchierte Patentansprüche: -
Nicht recherchierte Patentansprüche: 6,7
Grund für die Beschränkung der Recherche:

Verfahren zur therapeutischen Behandlung des menschlichen oder tierischen Körpers

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Berlin | 15-11-1979 | FROELICH |